Europäisches Patentamt

European Patent Office

Office européen des brevets

(19)

(11) Publication number: **0 155 433**

**A1**

(12) **EUROPEAN PATENT APPLICATION**

(21) Application number: **84810140.8**

(22) Date of filing: **23.03.84**

(51) Int. Cl.⁴: **C 12 P 21/00**
**A 61 K 37/02**
**//(C12P21/00, C12R1:91)**

(43) Date of publication of application
**25.09.85 Bulletin 85/39**

(84) Designated Contracting States:
**CH LI**

(71) Applicant **Fontana, Adriano**
**Section of Clinical Immunology Dept. of Internal**
**Medicine University Hospital Haldeliweg 4**
**CH-8044 Zürich(CH)**

(72) Inventor: **Fontana, Adriano**
**Section of Clinical Immunology Dept. of Internal**
**Medicine University Hospital Haldeliweg 4**
**CH-8044 Zürich(CH)**

(74) Representative: **Norris, Allen Edwin**
**SANDOZ AG Patentabteilung**
**CH-4002 Basel(CH)**

(54) **Immunosuppressant factor.**

(57) An immunosuppressant factor, a neuroblast growth inhibitory factor and an interleukin 1 like factor derived from human glioblastoma cells.

EP 0 155 433 A1

## IMMUNOSUPPRESSANT FACTOR

The present invention is concerned with immunomodulatory substances.

It is more particularly concerned with an immunosuppressant factor capable of inhibiting T-cell mechanisms particularly those which are Interleukin 2 (IL-2) dependent.

The role of T-cells in cell-mediated immunity e.g. by cooperation with β-lymphocytes is well established and substances which are capable of inhibiting T-cell mechanisms involved in the generation of antibodies would be used to suppress or reduce the body's immune response. Such substances could thus be employed e.g. in connection with transplants to prevent rejection and also in connection with the treatment of diseases characterised by an auto-immune response in the body.

We have now found that cultured human glioblastoma cells secrete a factor that inhibits interleukin 2 (IL-2) dependent T-cell mechanisms. This factor has an inhibitory effect on IL-2 induced proliferation of T-cell clones and on the induction of alloreactive cytotoxic T-cells in mixed lymphocyte cultures. It further inhibits the growth of neuroblasts but not of fibroblasts.

This factor can be obtained form the cystic fluid or SN of cultured human glioblastoma cell lines and has an approximate molecular weight of 97,000.

Cultured human glioblastoma cells are characterized by ultrastructural features, especially the presence of microfibrils, and by biochemical markers, such as glial fibrillary acidic protein (GFA) and S-100 protein (7, 14, 15). In addition human glioma cells express neurooctodermal antigens shared with melanomas and neuroblastomas (8), as well as la-like antigens and the common acute lymphoblastic leukemia antigen (CALLA), an antigen expressed on lymphoid cells from patients with the common form of acute lymphoblastic leukemia (16, 17).

Two immunoregulatory mediators, a suppressor factor and a helper factor, were detected in the supernatant of glioblastoma cells. The helper factor shares the characteristics of macrophage-derived IL 1. The characterization of the factor as an IL 1-like mediator is based on the finding that the factor a) enhances the PHA-induced thymocyte proliferation, b) exhibits no IL 2 activity, but c) augments the IL 2 production by nitrogen-stimulated spleen cells, and d) has a m.w. of 22,000. In this context it is of interest that mouse astrocytes stimulated with lipopolysaccharide and rat $C_6$ glioma cells secrete analogous IL 1-like factors in vitro (9, 18, 19). Furthermore, rat astrocytes have been shown to present antigen to T-lymphocytes, e.g. to activate myelin basic protein-specific T lymphocyte lines (20). In addition, cultured rat astrocytes express Ia antigens upon interaction with T cells (20). The production of IL 1-like factors and the presentation of antigen by astrocytes may have a central role in the generation of immune responses in the brain. There is also recent evidence that the central nervous system is capable of mounting specific immune responses to antigens and tumor cells when introduced to the brain (21).

Analogous to astrocytes, glioblastoma cells, which may represent transformed astrocyte precursor cells, do produce IL 1-like factors in vitro. The effect of the IL 1-like helper factor, however, is overriden by the presently described inhibitory factor also elaborated by the tumor cells. This factor inhibits the proliferative response of T cells to Con A or to both PHA and IL 1/IL 2 standards. The factor also blocks the proliferation of an H-2-restricted, hapten-specific T cell line that normally grows in the presence of IL 2 and haptenated irradiated spleen cells. The glioblastoma-derived suppressor factor has no effect on IL 2 production by Con A-stimulated spleen cells and does not influence the growth of a thymoma cell line (EL 4 cells), which proliferate independently of IL 2. However, the factor abrogates the IL 2-induced proliferation of a cloned T cell line. IL 2 does not substitute for the inhibitor effect of the supernatant containing the factor on the deveopment of CTL. Taken together, these results indicate that the factor interferes with terminal events in the T cell activation cascade. The hypothesis that this glioblastoma-derived suppressor factor influences proliferative steps not necessarily involving direct IL 2 effects on T cells is based on the finding that the 97,000-m.w. factor does also inhibit the growth of neuroblasts, which grow independently of IL 2.

Recently, the presence of immunosuppressive factors in the conditioned medium of various human tumor cells was investigated (22). Out of 12 different tumors (three melanoma, two sarcoma, and seven carcinoma) the SN of only three tumors produced greater than 30% inhibition of PHA-induced blastogenesis. However, as shown by dilution experiments the inhibitory effect was only visible up to a 1/4 dilution of the SN; higher dilutions had no effect (22). When using the conditions to produce and to test the globalstoma SN, the SN of two neuroblastoma, a melanoma, and a rhabdomyo-sarcoma had no effect on responsiveness of thymocytes to Con A. This supports the assumption that among nonlymphoid tumors glioblastoma cells are at the present time unique for their capacity to release immunosuppressive factors in vivo and in vitro.

Depression of immune responsiveness has been documented in patients with glioblastoma (1-6). Impared cell-mediated immunity in glioblastoma patients is indicated by depressed skin reactivity to common antigens, decreased ability to become sensitized to dinitrochlorobenzene, and decreased in vitro lymphocyte responsiveness to PHA or antigens. Factors released by the tumor cells may well account for the described T cell immune deficiency state. This idea is supported by the detection of imhibitory factors on T cell activation present in glioblastoma culture SN in vitro and the cyst fluid of glioblastoma tumors in vivo. In addition the sera of globalstoma patients contain a factor that inhibits the mixed lymphocyte reaction and the PHA- and Con A-induced lymphocyte proliferation in vitro. After removal of the glioblastoma by neurosurgery, suppressor activity in the scra disappeared (1, 4).

Although it is well documented that in most glioblastoma patients, humoral immune responses to their tumors develop (23, 24), there is little evidence of significant cellular anti-tumor immune responses (25,-27). In general, cytotoxic T cells have been implicated to contribute to the development of the cellular anti-tumor immune reactions (28, 29). The IL 2 dependency of the generation of CTL has been demonstrated by in vivo administration of highly purified IL 2 in concert with injections of tumor cells, which resulted in an augmented CTL and natural killer cell response in mice (30). The observed blocking of the generation of CTL by the glioblastoma cell-derived 97.000-m.w. factor according to the invention may account at least in part for inefficient immunosurvelliance against glioblastomas.

Impaired host immunocompetence may also emanate from lymphokine-induced production of mucopolysaccharide coats by glioblastoma cells that nonspecifically suppress the cellular immune response (31).

In addition to its relevance for modulation of T cell activation the supernatant containing the factor is of interest for its neuroblast growth inhibitory factor (NGIF)-like activity. NGIF is released by fetal rat glioblasts and exerts suppressor activities on neuroblasts but not on fibroblasts (31, 32). In addition, this 75.000-m.w. factor possessed the activity to promote morphological differentiation of Neuro 2A cells as evidenced by the formation of neural processes (32, 33). In an analogous assay we also observed that the Sephacryl S-200 glioblastoma fractions, which inhibited T cell responsiveness to IL 2 and the growth of neuroblasts, did induce neurile outgrowth.

References referred to in the specification:

1. W.H. Brooks, et.al., Depressed cell-mediated immunity in patients with primary intracrantal tumors. J. Exp. Med. 136:1631.

2. W.H. Brooks, et.al., Immune responses in patients with gliomas. Surg. Neurol. 2:419.

3. D.G.T. Thomas, et.al., Impaired cell-mediated immunity in human brain tumors. Lancet 11:1389.

4. H.F. Young, et.al., Inhibition of cell-mediated immunity in patients with brain tumors. Surg. Neurol. 5:19.

5. C.B. Menzies, et.al., Impaired thymus-derived lymphocyte function in patients with malignant brain tumor. Clin. Neurol. Neurosurg. 82:157.

6. T.L. Roszman, et.al., Immunobiology of primary intracrantal tumors. Suppressor cell function and lectin-binding lymphocyte subpopulations in patients with cerebral tumors. Cancer 50:2173.

7. S.C. Discrens, et.al., Characterisation of an established human malignant glioma cell line: LN-18. Acta Neuropathol. 53:21.

8. S.N. Carrel, et.al., Expression of neuroectrodernal antigens common to melanomas, gliomas and neuroblastomas. Identification by monoclonal antimelanoma and antiglioma antibodies. Acta Neuropathol. 57:158.

9. S.F. Fontana, et.al., Production of prostaglandin E and an interleukin 1-like factor by cultured astrocytes and C glioma cells. J. Immunol. 129:2413.

10. H.P. Pircher, et.al., Restriction fine specificity of longierm, hapten-specific cytotoxic T-cell clones: Analysis with H-2K mutant mice and H-2K -specific monoclonal antibodies. Eur. J. Immunol.

11. J.C. Cerottini, et.al., Cell-mediated cytotoxicity, allograft rejection, and tumor immunity. Adv. Immunol. 18-67.

12. J.T. Kung, et.al., Suppression of in vitro cytotoxic response by macrophages due to induced arginase. J. Exp. Med. 146:665.

13. B.M. Stalder, et.al., Relationship of cell cycle to recovery of IL-2 activity from human mononuclear cells, human and mouse T cell line. J. Immunol. 127-1936.

14. A. Pactau, et.al., Glial fibrillary acidic protein and intermediate filaments in human glioma cells. Acta Neuropathol. 47:71.

15. A. Edstrom, et.al., Morpholigical alterations and increase of S-100 protein in cultured human glioma cells deprived of serum. Exp. Cell Res. 83:426.

16. S. Carrel, et.al., Expression of HLA-DR and common acute lymphol lymphoblastic leukemia antigens on glioma cells. Eur. J. Immunol. 12:354.

17. N. de Tribolet, et.al., In progress in Experimental Tumor Research, Vol. 27, Brain Tumor Biology. M. I. Rosenblum, et.al., cds. Karger, Basel.

18. A. Fontana, et.al., Biological and biochemical characterization of an interleukin-1 like factor from rat C glioma cells. Euro. J. Immunol. 13:685.

19. A. Fontana, et.al., Dual effect of glia maturation factor on astrocytes: differentiation and release of interleukin-1 like factors. J. Neuroimmunol.

20. A. Fontana, et.al., Astrocytes present myclin basic protein in encephalitogenic T cell lines: a step in multiple sclerosis plaque formation. Nature.

21. M. Hasek, et.al., Immunological tolerance and tumor allografts in the brain. Nature 186:68.

22. C.M. Renk, et.al., Inhibition of normal allogeneic lymphocyte by a factor released from human tumor cells in culture. Cancer Immunol. Immunother. 11:7.

23. M. Pfreundschuh, et.al., Scrologic analysis of cell surface antigens of malignant human brain tumors. Proc. Natl. Acad. Sci. USA 75:5122.

24. H.B. Coakham, et.al., Autologous humoral response to human gliomas and analysis of certain cell surface antigens: in vitro study with the use of interocytotoxicity and immune adherrence assay. JNC1 64:223.

0155433

25. N.L. Levy, 1978. Specificity of lymphocyte-mediated cytotoxicity in patients with primary intracrantal tumors. J. Immunol. 121:903.

26. K.M.A. Sheik, et.al., Specific cellular immune responses in patients with gliomas. Cancer Res. 39:1733.

27. M.K. Gately, et.al., In vitro studies on the cell-mediated immune response to human brain tumors. Requirement for third-party stimulator lymphocytes in the induction of cell-mediated cytotoxic responses in allogenic cultured gliomas. JNC1 69:1245.

28. G.B. Millis, et.al., Generation of cytotoxic lymphocytes in syngeneic tumors by using co-stimulator (interleukin 2): in vivo activity. J. Immunol. 125:1904.

29. M.A. Cheever, et.al., Specific adoptive therapy of established leukemia with syngeneic lymphocyte sequentially immunized in vivo and in vitro and nonspecifically expanded by culture with interleukin 2. J. Immunol. 126:1318.

30. S.H. Heffeneider, et.al., In vivo interleukin 2 administration augments the generation of alternative cytolytic T lymphocytes and resident natural killer cells. J. Immunol. 130:222.

31. S.J. Dick, et.al., Lymphoid cell-glioma cell interaction enhances cell coat production by human gliomas: novel suppressor mechanism. Science 220:739.

32. T. Kato, et.al., 1982. Inhibition of growth of mouse neuoblastoma cells by protein factor derived from rat glioblasts. Dev.Brain Res. 3:645.

33. Y. Sakazaki, et.al. 1983. Characterization and partial purification of neuroblastoma growth inhibitor factor from the culture medium of glioblasts. Brain Res. 262:125.

The following examples illustrate the invention. The following abbreviations are employed:

IL 1, Interleukin 1; IL 2, Interleukin 2; NGIF, neuroblast growth inhibition factor; SN, supernatant; GFA, glial fibrillary acidic protein; DMEM, Dulbecco's modified Eagle's medium; ConA, concanvalin A; 2-ME, 2-mercaptoethanol; $^3$H-Tdr.[$^3$H]thymidine; PHA, phytohemaglutinin; OVA cells, ovalbminspecific T-cell line; CTL, cytotoxic T-lymphoctes; MLC mixed lymphocyte culture; LPS, Lipopolysaccharide; AED, N-idodoacetyl-N-[5-sulfonic-1-naphthyl]ethylene diamine; CH, cycloheximide.

Example 1

Four human glioblastoma cell lines are established and maintained in mono-layer cultures by the method described in Diserens et.al., Acta Neurophthol. 53:21. Four cells lines used in the examples are designated Cl-18; Cl-229; 992 and 308 and were maintained for 72, 28, 8 and 4 months respectively.

Characterization of the glioblastoma cell lines revealed that cells from Cl-229, 308 and 992 expressed la-like antigens on their surface, cells from 308 and 992 were positive for glial fibrilliary acidic protein (GFA), and cells from Cl-18, 308 and 992 expressed a neuro-ectodermal antigen identified by recently described monoclonal antibodies (Acta Neuropathol. 57:158 Carrell S., et.al.)

For production of SN the glioblastoma cells were plated at 1 x 10$^4$ to 1 x 10$^6$ cells/well (2.4 x 1.7 cm; Linbro Scientific) in culture medium consisting of Dulbecco's modified Eagle's medium (DMEM), 10% fetal calf serum (FCS), and 300 ug/ml L-glutamine. Twenty-four hours after plating the glioblastoma cells, the medium was replaced with 0.5 ml culture medium. The SN were collected 1 to 5 days later, centrifuged (2000 x G. 10 min) and ultrafiltrated by using a micropartition system with YM3 membrane (MPS.1, Amicon). The material deposited after centrifugation (1500 x G. 15 min) on the membrane (m.w. > 10.000), was resuspended in RPMI 1640 to give the original volume and passed through 0.45-μm filters.

Example 2 : Thymocyte proliferation assay

The effect of glioblastoma SN on the thymocyte proliferative response to concanavalin A (Con A) was tested in the following way. Samples of 50 $\mu$l of SN at various dilutions were added to $6 \times 10^5$ thymocytes from $C_3H$/HeJ mice: thymocytes were suspended in 150 ul of RPMI 1640 medium supplemented with 300 ug/ml L-glutamine. $1 \times 10^{-5}$ M 2-mercaptoethanol (2-ME). and 5% FCS in flat-bottomed microtiter plates and incubated for 72 hours in the presence of Con A (1 $\mu$g/well). Sixteen hours before harvest 0.5 $\mu$Ci of $^3$H-Tdr. 5.0 Ci/mmol) was added per well.

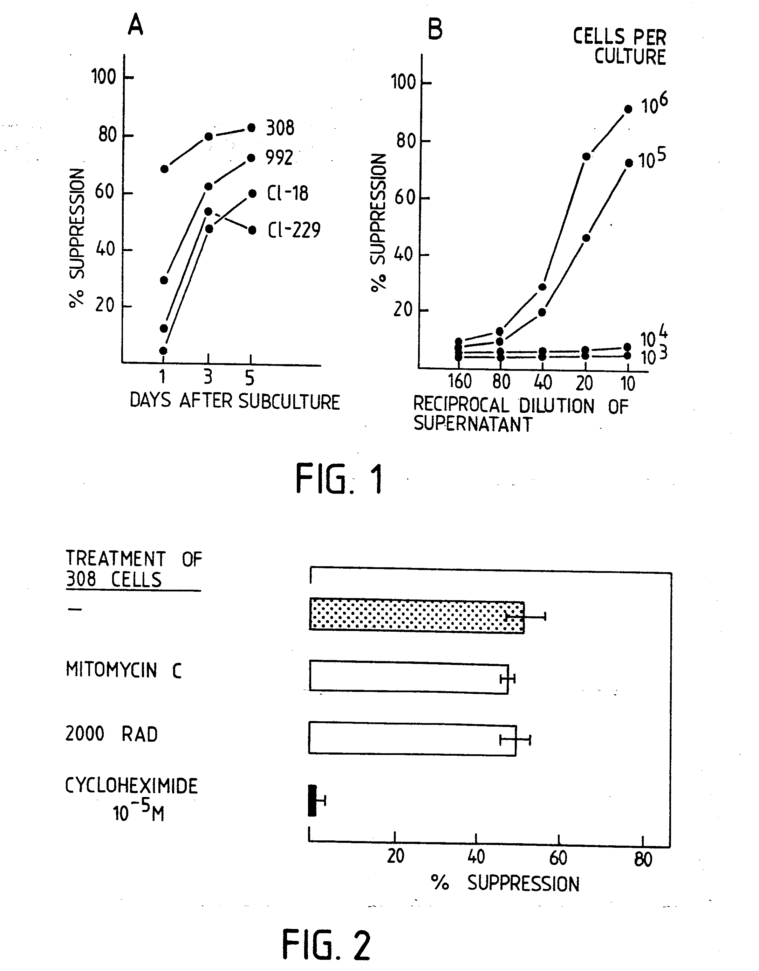

The results are shown in Figures 1A and B as percent suppression compared with the Con A response of thymocytes treated with a medium control.

In Figure 1A the SN of the four glioblastoma cell lines used were harvested after 1, 3 and 5 days and tested at a final dilution of 1/10 on the thymocytes.  In Figure 1B the testing took place on SN of 308 cells harvested  after culture for 5 days at different seeding densities and final dilutions.  In the absence of glioblastoma SN or medium control the background count was $483^\pm$ 229 and the Con A response $76.797^\pm 6.631$ cpm.

The magnitude of inhibitory potency of the SN can be seen to be dependent on time of culture and cell density.

In selected experiments on metabolic inhibition using 308 cells the production of SN was performed with irradiated (2000 R) or mitomycin C-treated (50 $\mu$g/ml, 30 min. 37°C) glioblastoma cells. Alternatively cycloheximide ($10^{-5}$M) was added to glioblastoma cells at the beginning of the 72-hour culture; cycloheximide was also added to control SN collected after 72 hours. These SN were ultrafiltrated three times before testing to remove CH.

The results of these experiments are summarised in Figure 2, where the SN of $10^6$ cells cultured over 5 days were employed. As a CH control the SN of CH treated 308 cells and of 308 SN supplemented with CH after collection were untrafiltrated.

In a further control, experiments were carried out on four established human tumor cell lines unrelated to glioblastoma cells. These results are summarised in Table 1.

### TABLE I

Effects of SN from various tumor cell lines on lymphocyte blastogenesis

| Type of Malignancy [a] | Designation | Con A Response [b] (cpm ± SD) | % Suppression |
|---|---|---|---|
| Neuroblastoma | SR.N.SH | 52.123 ± 1.817 | 6.0 |
| Neuroblastoma | IMR.32 | 50.041 ± 2.751 | 9.8 |
| Melamoma | Me.43 | 58.382 ± 447 | 0 |
| Rhabdomyosarcoma | RD | 51.720 ± 904 | 6.8 |

[a] Tumor cells were cultured at a seeding density of $10^6$ cells/ml for 5 days.

In the presence of Con A, thymocytes were incubated with 1/10 dilution of tumor cell SN or a control culture medium for the tumor cell SN. In the presence of medium control the background count of thymocytes was 432 ± 112 and the Con A response was 55441 ± 4817.

It will be noted that the release of immunosuppressive factors does not appear to be a general property of cultured tumor cells.

Analysis of the L-arginine content in 308 SN revealed that inhibition is not due to arginine degradation in the medium by an arginase release by 308 cells. Furthermore, the 308-mediated inhibition could not be explained by the production of interferon as no antiviral activity was detected in 308 SN.

Example 3  Effect of glioblastoma SN on B lymphocytes and non-lymphoid cells

Mouse fibroblasts (A9, 3T3, LS cell lines) were cultured in DMEM supplemented with L-glutamine (300 ug/ml) and 10% FCS: mouse neuroblasts (Neuro 2A, NB4, 1A3 cell lines) were plated in Earle's minimum essential medium with 10% FCS. L-glutamine (300 ug/ml), and 1% nonessential amino acids (100x). Fibroblasts and neuroblasts ($10^4$ cells/well) were incubated in 0.2 ml of medium for 72 hours. For the final 6 hours, the cells were pulsed with 1.2 uCl of $^3$H-Tdr. Thereafter, the medium was aspirated from each well, replaced with trypsin-EDTA. and incubated (37°C, 10 min). Finally, the cells were harvested with the use of an automated harvester.

The data in Table II indicate that when compared with its effect on thymocytes, the 308 SN was less inhibitory on B cell proliferation: the percentage of inhibition of the LPS-induced stimulation of spleen cells was 29%, whereas the corresponding value for the mycloma cell line X63-Ag8 was 21%.

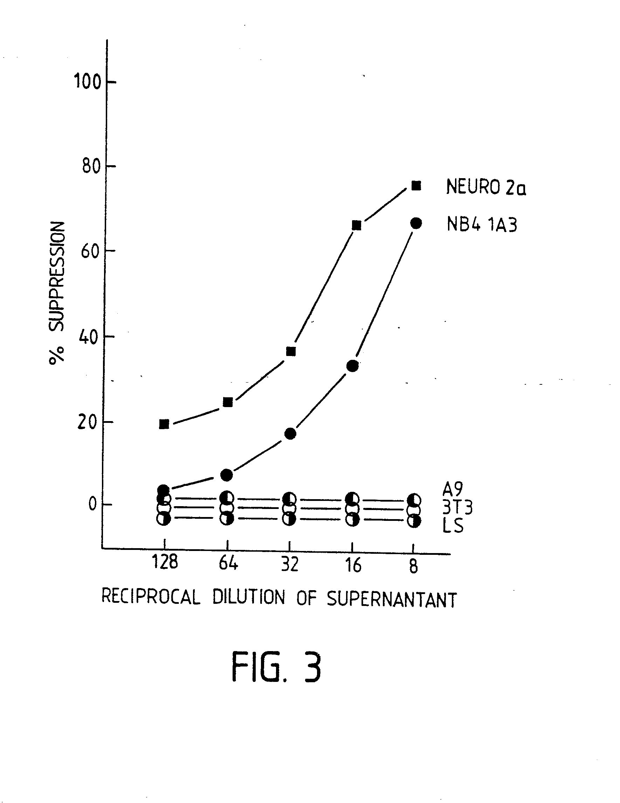

There was no difference in thymidine uptake between fibroblast cultures containing 308 SN and those being supplemented with a medium control. This was true for all three fibroblast cell lines tested. In contrast, the gliobalstoma SN singificantly suppressed the growth of the two neuroblast cell lines as measured by the uptake of $^3$H-Tdr (Fig.3, Table II) and by counting the number of cells at termination of the 72 hr cultures. Twenty-four, 48 and 72 hr after culturing 1 x $10^4$ Neuro 2A cells/well, the cell numbers x $10^4$/well of quadruplicate cultures were in the presence of the 308 SN: 98$^+_-$ 0.17 (24 hr),1.80$^+_-$ 0.36 (48 hr), and 4.3$^+_-$0.44 (72 hr), whereas the corresponding values for the medium controls were 0.88$^+_-$0.05, 2.55$^+_-$0.06, and 6.08$^+_-$0.40, respectively.

## TABLE II

### Effect of 308 SN on lymphoid and nonlymphoid cells

| Cell Type | Description or Strain | Stimulant | $^3$H-Tdr Uptake(cpm) | | |
| --- | --- | --- | --- | --- | --- |
| | | | Medium control | 308 SN[a] | % Suppression |
| Thymocytes | C3H/HeJ | Con A | 66.709 $\pm$ 3.104 | 8.323 $\pm$ 235 | 88 |
| T cell lymphoma | EL-4 | - | 137.960 $\pm$ 25.173 | 164.487 $\pm$ 6.253 | 0 |
| Spleen cells | DBA | LPS[b] | 72.442 $\pm$ 3.886 | 50.990 $\pm$ 2.184 | 29 |
| Myeloma cells | X63-Ag8[b] | - | 117.090 $\pm$ 2.597 | 92.580 $\pm$ 6.351 | 21 |
| Neuroblasts [c] | NB41A3 | - | 48.073 $\pm$ 1.756 | 15.504 $\pm$ 833 | 68 |
| Neuroblasts | Neuro 2A | - | 250.807 $\pm$ 19.700 | 62.408 $\pm$ 6.108 | 76 |
| Fibroblasts[c] | A9 | - | 78.591 $\pm$ 11.029 | 78.323 $\pm$ 10.444 | 0 |
| Fibroblasts | 3T3 | - | 117.316 $\pm$ 8.484 | 117.011 $\pm$ 4.026 | 0 |
| Fibroblasts | L929 | - | 84.395 $\pm$ 6.189 | 92.391 $\pm$ 5.674 | 0 |

[a] All assays were performed with the same 308 SN added to give a final dilution of 1/10.

[b] After lysis of erythrocytes 6 x 10$^5$ spleen cells were stimulated with lipopolysaccharide (LPS.E.coli 0127 B8, Difco Lab.). The mouse X63-Ag8 myeloma cells were cultured at 10$^4$ cells/well in DMEM supplemented with 10% FCS.

[c] See legend of Figure 3.

In Figure 3 the SN of 10$^6$ cells cultured for 5 days were employed together with a medium control.

<u>Example 4</u>   Mechanisms of glioblastoma SN-induced inhibition of T-cell activation

Thymocytes were treated with 308 SN (final dilution of 1/10) in the presence of PHA (0.5 ug/well) and various dilutions (1/10 in 1/160) of IL 1 or IL 2. In the absence of interleukins the background count was 209 $\pm$ 84 and the PHA response was 3074 $\pm$ 425.

The IL 1 preparation used was prepared from LPS-stimulated peritoneal cells with Con A-Sepharose as described in reference 7.

The results are summarised in Table III.

TABLE III

Effect of 308 SN on thymocytes stimulated with PHA and various doses of IL 1 or IL 2

| Stimulant [a] | | $^3$H-Tdr Uptake (cpm) | | % Suppression |
|---|---|---|---|---|
| | | Medium control | 308 SN | |
| PHA + IL 1 [c] | 1/10 | 76.342 + 5.978 | 12.104 + 618 | 84.2 |
| | 1/20 | 69.831 + 5.150 | 13.423 + 212 | 81.8 |
| | 1/40 | 42.143 + 1.706 | 9.061 + 842 | 78.5 |
| | 1/80 | 18.407 + 128 | 2.648 + 221 | 85.7 |
| PHA + IL 2 [b] | 1/10 | 84.457 + 3.621 | 13.551 + 817 | 84.0 |
| | 1/20 | 84.882 + 4.185 | 15.492 + 1.005 | 81.8 |
| | 1/40 | 76.537 + 2.404 | 11.250 + 1.423 | 85.4 |
| | 1/80 | 43.244 + 1.753 | 5.973 + 175 | 86.2 |
| | 1/160 | 26.505 + 1.044 | 3.182 + 3.279 | 88.0 |

[a] Thymocytes were treated with 308 SN (final dilution of 1/10) in the presence of PHA (0.5 µg/well) and various dilutions (1/10 to 1/160) of IL 1 or IL 2. In the absence of interleukins the background count was 209 + 84 and the PHA response was 3074 + 425.

[b] The IL 1 preparation used was prepared from LPS-stimulated peritoneal cells of BALB/c mice; the IL 2 was prepared by stimulation of BALB/c spleen cells with Con A-Sepharose as described previously (17).

These results show that inhibition of the Con A response of thymocytes apparently results from influences on late events of the T cell activation cascade, because the 308 SN inhibited the proliferative response of thymocytes stimulated with PHA in the presence of IL 1 or IL 2. The degree of 308 SN-induced suppression of proliferation was similar at various concentrations of IL 1 or IL 2 employed.

0155433

EXAMPLE 5:

Effect of SN-308 on cloned T-cell lines

Proliferation of $H-2^b$-restricted hapten (N-iodoacetyl-N-(5-sulfonic-1-naphthyl) ethylene diamine [AED])-specific cloned cytotoxic T cells (CTL) (10) was assessed in culture medium consisting of Iscove's modified DMEM supplemented with 10 % FCS, 1 x $10^5$ M 2-ME, antibodies, and 10 % (v/v) of a 24-hr spleen cell Con A SN (IL 2 SN). The CTL ($10^4$/well) were cultured alone or together with $10^6$ irradiated (2000 rad) haptenated spleen cells ($H-2^b$) as stimulators. Various dilutions of SN of 308 glioblastoma cells or of a control medium were added to the mixture of the CTL and stimulators cultured in culture medium for 72 hr. Sixteen hours before harvest, each well was pulsed with 1.2 $\mu$Ci of $^3$H-Tdr.

308 glioblastoma SN was also tested on cloned ovalbumin-specific T cells (OVA cells). The OVA cells were cultured for 48 hr in 0.2 ml culture medium (see above) with IL 2 SN. The IL 2-dependent proliferation was tested in the presence of various dilutions of 308 SN or medium control. The incorporation of $^3$H Tdr (1.2 $\mu$l/well) was measured over a 12 hr period.

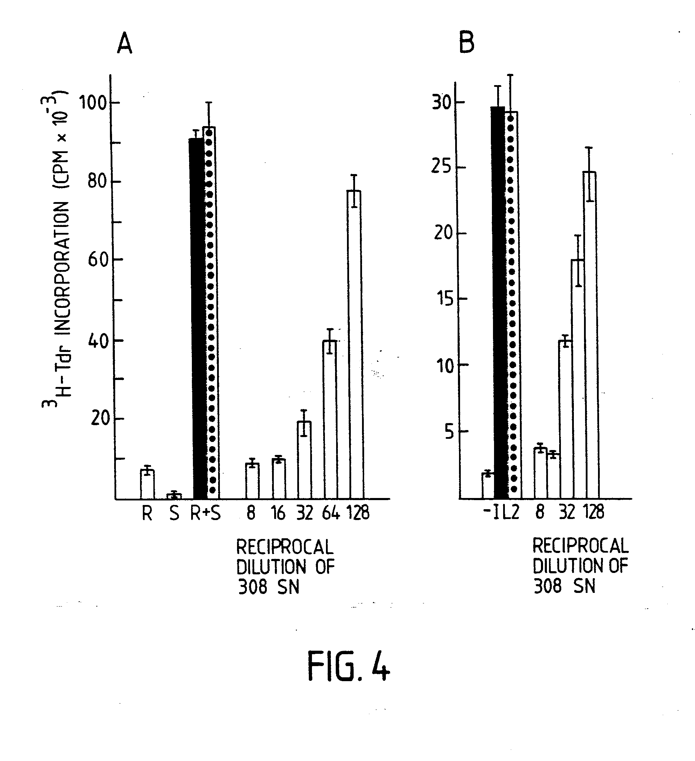

The results are shown in figures 4A and 4B. In A. hapten-specific cloned CTL were cultured (at $10^4$ cells) alone (R) or together with $10^6$ irradiated (2000 rad) AED-haptenated spleen cells as stimulators (S) (black bar). Various dilutions (1/8 to 1/128) of 308 glioblastoma SN or a 1/8 dilution of control medium ([ · · · · · ]) were added to the mixture of CTL and cultured for 72 hr. In B $10^4$ OVA cells were cultured for 48 hr with IL 2 SN (black bar) or without IL 2 SN (———). The 308 glioblastoma SN (1/8 to 1/128 diluted) or a 1/8 dilution of control medium ([ · · · · · ]) was added to OVA cells in the presence of IL 2 (final dilution 1/20). Data are given as counts per minute of $^3$H-Tdr incorporation.

Figure 4A shows that cells of an H-2-restricted, hapten-specific T cell line proliferated in the presence of IL 2 and haptenated, irradiated spleen cells as stimulator cells. This proliferation was completely blocked by adding the glioblastoma-produced factor. Maximal inhibition was noted when the factor was added in OVA T cells growing in the presence of IL 2 only (Fig. 4B).

From the experiments outlined above, the inhibition of T cell growth by glioblastoma cell-derived factors could be due to their direct interference with IL 2-triggered events leading to T cell proliferation. This possibility is supported further by the finding that the 308 SN had no effect on the growth of a thymoma cell line that grows of a thymoma cell line that grows independently of IL 2 (Table II).

EXAMPLE 6:

The release of analogous inhibitory factors by the glioblastoma cells in vivo was demonstrated by testing the ultrafiltrated cyst fluid of the patient from whom the 308 cells originated. The cyst fluid (final dilution 1/20), ultrafiltrated on YMB membranes, induced a 28% inhibition of the IL 2-induced proliferation of OVA T cells, whereas 12 control sera had no effect.

EXAMPLE 7:

Effect of glioblastoma SN on the induction of cytotoxic T-cells

Anti-$H-2^b$-specific CTL were induced by stimulation of 12 x $10^6$ B10.D2 ($H-2^d$) spleen cells with 30 x $10^6$ irradiated (2000 R) C57Bl/6 ($H-2^b$) stimulator spleen cells in 6 ml Iscove's modified DMEM containing 10 % FCS. 1 x $10^{-5}$ M 2-ME, and antibiotics (mixed lymphocyte culture (MLC) medium). After 6 days the specific cytotoxic activity of the CTL was tested in a 3-hr $^{51}$Cr-release

assay in 0.2 ml cultures containing $10^4$ $^{51}$Cr-labeled target cells EL-4 (H-$2^b$), P815 (H-$2^d$). Yac-1 (H-$2^d$)) at different target ratios as described in 11. The cytotoxic effect of the cells cultured over 6 days in MLC medium was compared with the effects of cells cultured in MLC medium being supplemented with medium control or 308 SN.

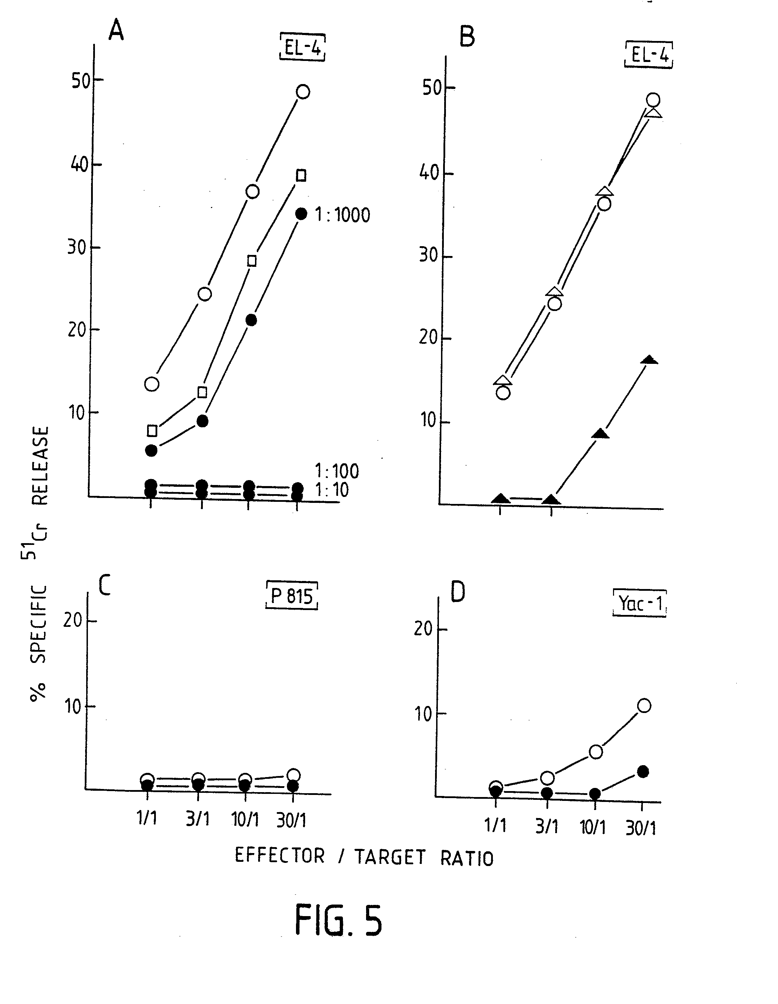

The results are summarized in Fig. 5A-D. Legend Fig. 5.
The cytotoxic effect of the cells cultured over 6 days in MLC medium (O-O) was compared with the effects of cells cultured in MLC medium being supplemented with a) medium control (final dilution 1/10) (□————□), b) 308 SN (●————●) diluted 1/10 (A-D). 1/100 or 1/1000 (A) or with c) Sephacryl S-200 column fractions that were known to inhibit (▲————▲) or to have no effect (△————△) on the IL 2-dependent proliferation of OVA T cells. The fractions to be assayed were concentrated 10 x on YMB membranes, and the concentrated fractions were added to MLC medium to give a final dilution of 1/10. Results are given as percent specific $^{51}$Cr release (see Reference 11). Test duration 3 hr. Mean values of three samples.

'The preparation and testing of Sephacryl S-200 column fractions is described below).

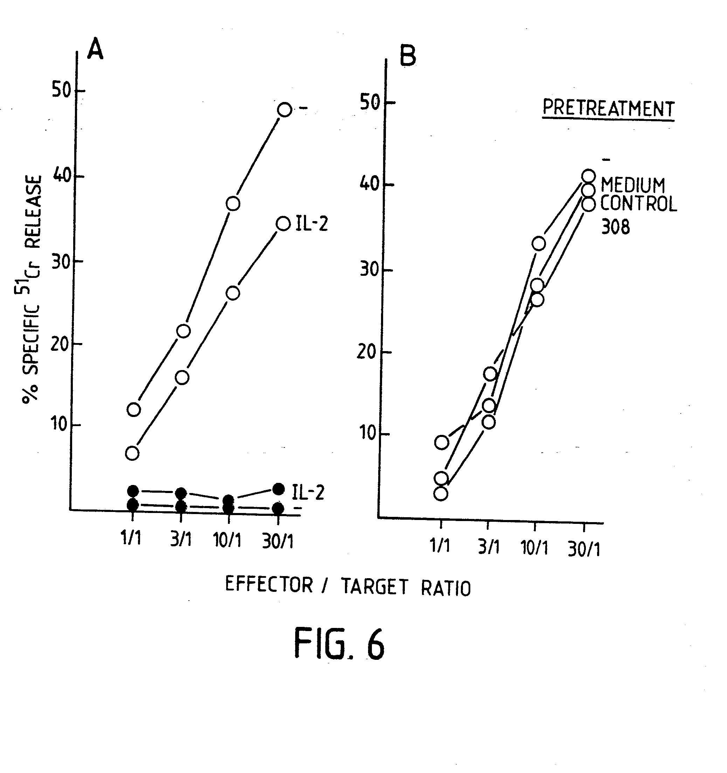

As shown in figure 6 the addition of IL 2 together with the 308 SN factor did not restore the generation of CTL. A 2-hr preincubation of mature CTL with 308 SN had no influence on the function of CTL on their targets.

In Fig. 6 A during the entire culture period of 6 days, IL 2 (final concentration of 10 % Con A SN) was present in MLC treated with 308 SN (1/10 diluted) (●————●) or medium control (O————O). After 6 days the CTL activity was tested on EL-4 cells. In B. before testing on EL-4 cells, CTL generated over 6 days in culture medium were preincubated for 2 hr in medium control or 308 SN (1/4 diluted).

EXAMPLE 8:

Biochemical characterisation of the glioblastoma SN

The 308 glioblastoma SN obtained from 308 cells cultured in serum free conditions over 96hr was concentrated 30-fold by ultra-filtration on YM-1C (Amteon) membrane, and 1.5 ml of concentrated SN was applied on a Sephacryl S-200 (column size 2.5 x 85 cm) at 4°C as described (9). The eluate collected in 2.5 ml fractions was monitored for adsorbance at 280 nm and was tested on fibroblasts (IL 929) and neuroblasts (Neuro 2 A) as described above. In addition the fractions were tested on OVA cells in the presence of IL2 and on thymocytes stimulated with PHA. The column, in a parallel run, was calibrated with y-globulin (y-Glob. m.w. 150.000). bovine serum albumin (BSA. m.w. 67.000), ovalbumin (OVA, m.w. 45.000), α-chymotrypsinogen (CHY. m.w. 25.000), and cytochrome c (CYT C. m.w. 12.400).

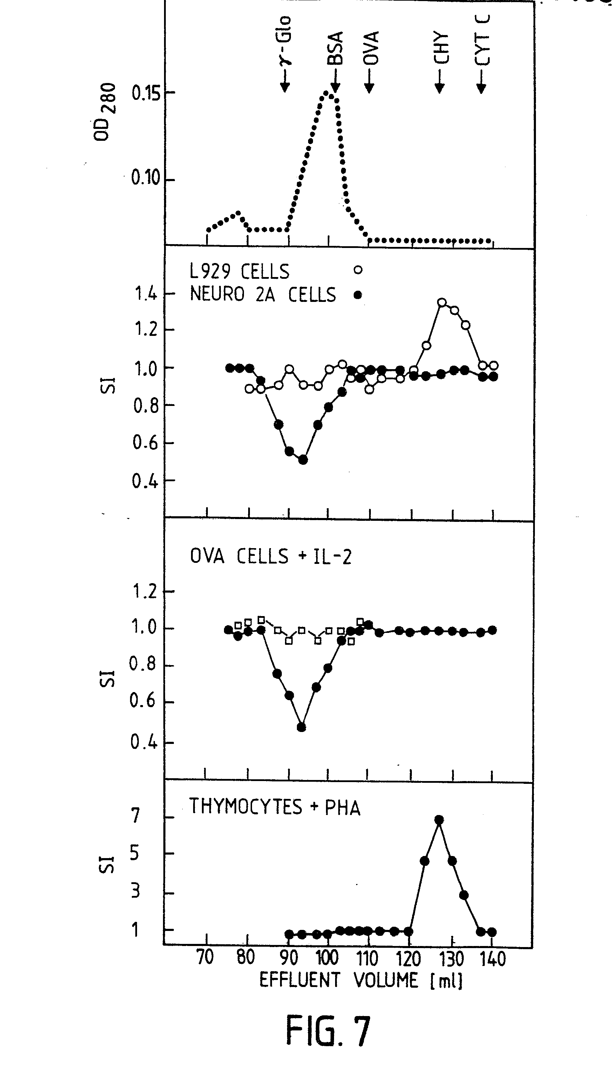

These results are summarized in figure 7 whereby the upper table shows the calibration run. Results are given as stimulation index (SI)comparing proliferation of cells treated with column fractions to cells treated with medium control. Results of serum-free control medium fractionated on a Sephacryl S-200 column are shown on OVA cells treated with II. 2 (□———□).

The peak of the inhibitory activity on neuroblasts and OVA cells was eluted from the column at the identical position, with an apparent m.w. of 97.000 (Fig. 7). Sephacryl S-200 fractions of a medium control consisting of DMEM (Fig. 7) and a medium control supplemented with 10 % FCS (data not shown) had no inhibitory effects on the growth of OVA cells stimulated with IL 2. Furthermore, no inhibitory effect was observed when testing 308 SN fractions on fibroblasts. In fact, the growth of fibroblasts but not of neuroblasts or of IL 2-treated OVA cells was augmented by fractions that exhibit an apparent m.w. of around 22.000. These fractions were identified to additionally stimulate the PHA response of mouse thymocytes (Fig. 7) but to have no effect on the growth of OVA cells cultured in the absence of IL 2: the incorporation of $^3$H-Tdr by OVA cells was $284 \pm 72$ and in the presence of IL 2 (final dilution 1/10) was $62038 \pm 2981$. When OVA cells were treated with 308 fractions eluting from the column between m.w. 150.000 and 10.000, no proliferation of the OVA cells was observed, with cpm values never exceeding 500. This indicates the absence of IL 2 in 308 fractions including those that were found to augment the PHA response of thymocytes.

EXAMPLE 9:

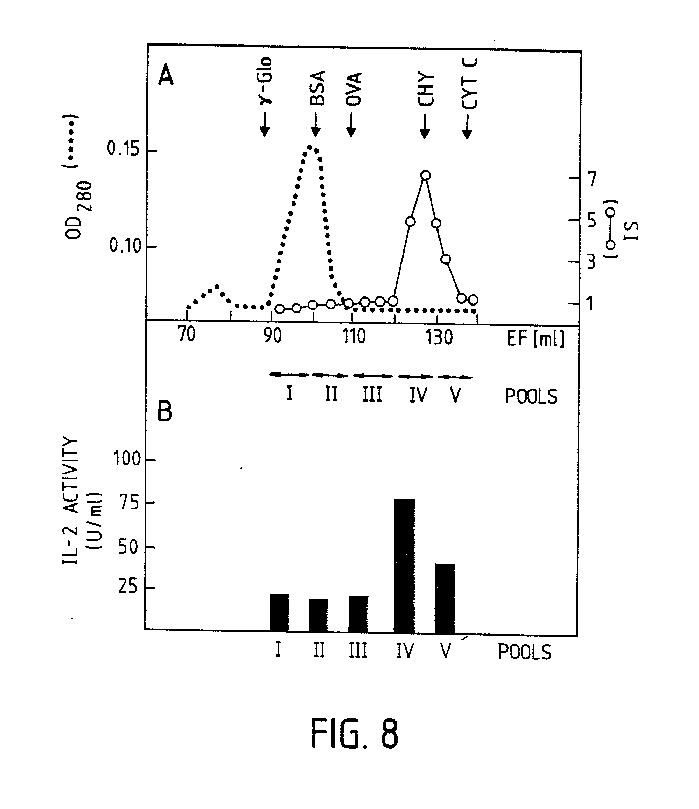

Pools of chromatographed fractions of the 308 SN were assayed for their ability to enhance the release of IL 2 by spleen cells treated with suboptimal doses of Con A. and the results summarized in Fig. 8.

In Fig. 8 A fractions were assayed for their capacity to augment the PHA response of thymocytes: data are given as stimulation index (SI) comparing the PHA response of thymocytes treated with column fractions to untreated thymocytes. EF, effluent volume (ml). In B, pooled fractions (pool I to V) were concentrated 10 x on YMB membranes and were tested for their ability to enhance the release of IL 2 by spleen cells ($5 \times 10^6$/ml). After 44 hr the spleen cell SN were harveated, and the IL 2 activity was assayed on OVA cells. The activity in test samples was transformed into units as described in reference 13.

Activity

$$= \frac{\text{Reciprocal titer of test sample at 30 \% of maximal standard cpm}}{\text{Reciprocal titer at 30 \% of maximal standard cpm}}$$

In the absence of pooled fractions of 308 SN the amount of IL 2 released by spleen cells treated with Con A (0.5 μg/ml) was 21 U /ml: upon treatment with optimal doses of Con A (5 μg/ml the IL 2 content measured was 97 U/ml.

Compared with Con A alone, pool I to III neither stimulates nor inhibited the release of IL 2 (Fig. 8). However, with fraction pool IV and V the increase of the amount of IL 2 released was 3.5- and 1.6-fold, respectively (Fig. 8). Thus the capacity to enhance the release of IL 2 by spleen cells appears to be associated with fractions augmenting the PHA response of thymocytes.

In addition to inhibiting neuroblast growth, the 308 fractions were found to induce the outgrowth of neurites of Neuro 2A cells: 66 % of Neuro 2A cells treated with fraction 93 (peak inhibitory fraction on neuroblast growth) showed cell processes, whereas the corresponding values in cultures treated with control fractions (fraction 112) or control medium were 21 and 22 %, respectively.

The immunosuppressant factor according to the invention is indicated for use in the treatment of diseases and conditions where suppression of the body's immune response is desired. Thus use in connection with transplant operations to prevent rejection is indicated. Examples of diseases where suppressions of the body's immune systems is indicated include the so-called auto-immune diseases.

The factor according to the invention can be employed in mixed form as purified supernatant from appropriate glioblastoma cell-lines or in fractionated and purified form as hereinbefore des-cribed.

It can be got up in suitable form for administration in a manner conventional for substances of this nature in accordance with the condition to be treated.

0155433

CLAIMS:

1. An immunosuppressant factor derived from human glioblastoma cells substantially as hereinbefore characterised.

2. A factor as claimed in claim 1 which inhibits interleukin 2 dependant T-cell mechanisms.

3. A factor as claimed in claim 1 having an approximate molecular weight of 97.000.

4. A factor for the inhibition of neuroblast growth substantially as hereinbefore characterised.

5. A factor as claimed in claim 4 having an approximate molecular weight of 75,000.

6. An interleukin 1 like factor derived from human glioblastoma cells substantially as hereinbefore characterised.

7. A factor as claimed in claim 7 having an approximate molecular weight of 22,000.

8. A supernatant harvested from cultured human glioblastoma cells containing a factor according to any one of claims 1 to 3 and/or claims 4 and 5 and/or claims 6 and 7.

9. The steps, features, compositions and compounds referred to or indicated in the specification and/or claims of this application, individually or collectively, and any and all combinations or any two or more of said steps or features.

- 1/7

0155433

FIG. 1

FIG. 2

FIG. 3

FIG. 4

FIG. 5

FIG. 6

**0155433**

FIG. 7

FIG. 8

**0155433**

. Application number

EP 84 81 0140

**European Patent Office**

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS/RRM, vol. 27, 1984, abstract no. 45949, Philadelphia, US; A. FONTANA et al.: "Immunodeficiency in glioblastoma patients, glioblastoma cells release factors inhibiting interleukin 2 mediated effects on T cells" & NEUROLOGY (USA) 1984, vol. 34, no. 3, suppl. 1, page 184 * title and "terms" * | 1-3 | C 12 P 21/00 A 61 K 37/02 // (C 12 P 21/00 C 12 R 1/91 ) |
| D,A | THE JOURNAL OF IMMUNOLOGY, vol. 129, no. 6, december 1982, pages 2413-2419, US; A. FONTANA et al.: "Production of prostaglandin E and on interleukin-1 like factor by cultured astrocytes and C6 glioma cells" * whole document * | 1-9 | |

TECHNICAL FIELDS SEARCHED (Int. Cl.4)

C 12 P
C 12 N
A 61 K

The present search report has been drawn up for all claims

| Place of search | Date of completion of the search | Examiner |
|---|---|---|
| THE HAGUE | 06-12-1984 | RYCKEBOSCH A.O.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82